# EUROPEAN PATENT APPLICATION

(11) **EP 2 578 261 A1**
(43) Date of publication of application: **10.04.2013**
(21) Application number: 11184403.1
(22) Date of filing: 07.10.2011
(51) Int. Cl.: A61M 5/42

(54) **Injection site indicator**

(71) Applicant: Sanofi-Aventis Deutschland GmbH, 65929 Frankfurt (DE)
(72) Inventor: The designation of the inventor has not yet been filed
(74) Representative: KEIL & SCHAAFHAUSEN Patentanwälte

(57) **Abstract**

Described is an injection site indicator (1, 6) comprising a plurality of targets (3) identifying a plurality of injection sites; and a unique label (5) adjacent to each of the plurality of targets (3).

## Description

The present invention relates to an injection site indicator.

### Background

An intraocular injection device may be used to administer therapeutic substances to eyes, such as eyes of mammals having eye disorders or diseases.

A number of vision-threatening disorders or diseases of the eye need to deliver a medicament (pharmaceutical, biological, etc.) and/or implantable device to a posterior segment of the eye by intraocular delivery (more specifically intravitreal delivery). One such technique for intraocular delivery is accomplished by intraocular injection into the vitreous body. A conventional apparatus for intraocular injection may include a pre-filled syringe of a medicament.

When a treatment protocol calls for a series of injections, previous injection sites may not be taken into account when administering subsequent injections. That is, injections may be delivered to the same injection site on more than one occasion, which can inhibit the healing process.

Therefore, there is a need for an injection site indicator which facilitates the administration of a series of injections.

### Summary of the Invention

It is an object of the present invention to provide for an apparatus that prevents multiple injections at the same site or at sites lying close together.

In an exemplary embodiment, an injection site indicator according to the present invention comprises a plurality of targets identifying a plurality of injection sites, and a unique label adjacent to each of the plurality of targets.

In an exemplary embodiment, the indicator further comprises an alignment feature corresponding to an anatomical feature. The alignment feature includes an eye hole. The plurality of targets is arranged in a predetermined pattern relative to the alignment feature.

In an exemplary embodiment, the label includes at least one of a number, a time, a day, a month, a year, a color, text, a graphic and a type of medicament.

In an exemplary embodiment, a given target of the plurality of targets includes a first state indicating that an injection has not been administered for the given target and a second state indicating that an injection has been administered for the given target.

In an exemplary embodiment, the plurality of targets is dye packs or perforated elements.

In an exemplary embodiment, the indicator is an eye mask or a chart.

The person skilled in the art understands that the present invention is not restricted to the explained possibilities.

The above mentioned advantages as well as other advantages of various aspects of the present invention will become apparent to those of ordinary skill in the art by reading the following detailed description, with appropriate reference to the accompanying drawings.

### Brief Description of the Drawings

Exemplary embodiments of the present invention are described herein with reference to the schematic drawings in which:
Figure 1 shows an exemplary embodiment of an injection site indicator according to the present invention; and
Figure 2 shows another exemplary embodiment of an injection site indicator according to the present invention.

### Detailed Description

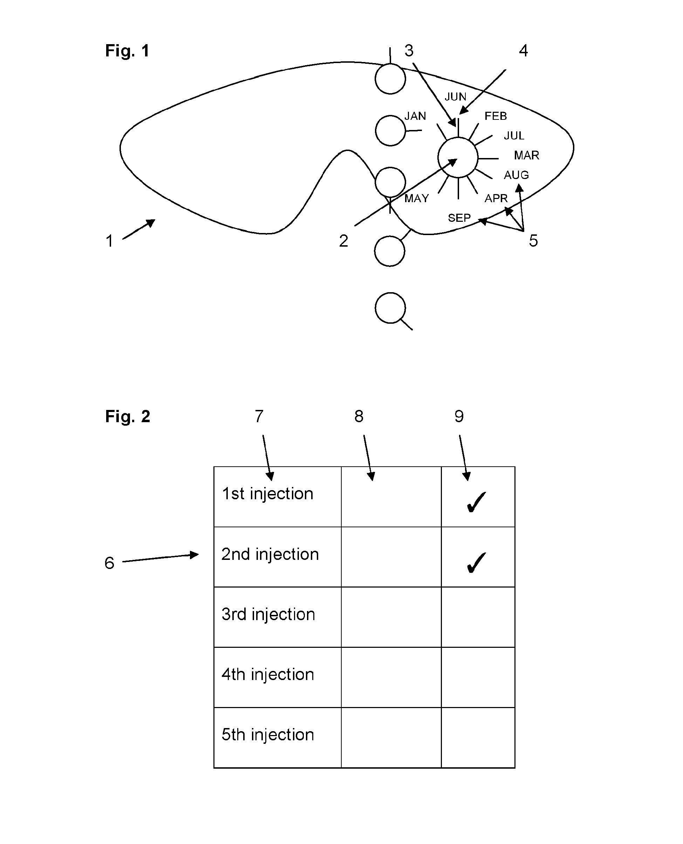

Figure 1 shows an exemplary embodiment of an injection site indicator 1 according to the present invention. In an exemplary embodiment, the indicator 1 is in the form of a template or mask which is adapted to indicate the position of a series of intravitreal injections. The indicator 1 has a shape and size to fit an injection site. For example, for intravitreal injections, the indicator 1 may be sized and shaped as an eye mask, similar to those placed over the eyes for darkening. An alignment feature (e.g., eye hole 2, a nose bridge portion, etc.) may be used to align the indicator 1 on the injection site. In the exemplary embodiment shown in Figure 1, the indicator 1 may include one or two eye holes 2 for alignment with the eye or a portion of the eye (e.g., the cornea). Thus, if a patient wears the indicator 1, one of the eyes is at least partly visible through eye hole 2.

In an exemplary embodiment, the indicator 1 is provided with a plurality of injection site targets 3 which indicate positions for administering injections. In an exemplary embodiment, the targets 3 may be rays extending in a radial direction from the eye hole 2. A distal end 4 of each ray defines a discrete location on the eye under the indicator 1. Thus, the series of rays and constitute a series of injection sites on the eye. In another exemplary embodiment, the targets 3 may be a constellation of holes or other geometric shapes to identify the injection sites. The targets 3 may be arranged relative to the alignment feature (e.g., the eye hole 2) so that the injection sites do not overlap and are adequately spaced from each other to prevent repeated injection (unless desired) on the same injection site. The spacing and alignment of the targets 3, in conjunction with the alignment feature, may also direct the injection so that the correct anatomical target is reached (e.g., the vitreous body) and other anatomical targets are avoided (e.g., the cornea).

In an exemplary embodiment, each of the targets 3 includes a unique label 5. In an exemplary embodiment, the label 5 may be a number (e.g., 1, 2, 3, etc.), a time (e.g., 12:00pm, 6:00pm, etc.), a day (e.g., Monday, Tuesday, etc.), a month (January, February, etc.), a year (e.g., 2011, 2012, etc.) or any combination thereof. In another exemplary embodiment, the label 5 may be color-coded and/or include text or a graphic. For example, the color green may be associated with a first medicament and the color yellow may be associated with a second medicament.

In an exemplary embodiment, the targets 3 may include a first state (prior to use) and a second state (after use). For example, the targets 3 may include perforated sections which are intact in the first state but are separate in the second state. In another exemplary embodiment, a change from the first state to the second state may be indicated by a color change. For example, the target 3 may be a dye pack which is pierced when an injection is administered through the target 3. The dye may bleed into a portion of the indicator 1 adjacent the target 3 to indicate that the target 3 has been used.

Another exemplary embodiment of the injection site indicator is shown in Figure 2. In this exemplary embodiment, the indicator 6 is depicted in the form of a chart or a table. The indicator 6 has e.g. three columns a row for each injection. A first column 7 includes the labels 5. A second column 8 includes the targets 3. And, a third column 9 includes a space for indicating that an injection has been administered. For example, when an injection has been administered a patient or other person (e.g., physician) may place a mark in the third column 9 on the corresponding row.

In practice, a user of the injection site indicator 1 or 6 first identifies the appropriate target 3 for the next due injection. For example, the user may utilize the labels 5 or identify the targets 3 which remain in the first state to identify the appropriate target 3. After that, a user may puncture the eye at the injection site assigned to the present point in time. The targets 3, in an exemplary embodiment, may be adapted to receive a needle. Hence, the injection site indicators 1, 6 are suitable to indicate injection sites with a view to increasing the number of sites into which a number of injections may be administered over a course of repeated injections avoiding repeated injections into a very localized site.

Those of skill in the art will understand that in addition to preventing repeated injections at the same site, the exemplary embodiments of the present invention may further aid in compliance with treatment protocols. For example, a patient and/or physician may examine the indicator 1, 6 to determine whether the patient is following the treatment protocol.

Those of skill in the art will understand that modifications (additions and/or removals) of various components of the apparatuses, methods and/or systems and embodiments described herein may be made without departing from the full scope and spirit of the present invention, which encompass such modifications and any and all equivalents thereof.

## Claims

1. An injection site indicator (1, 6) comprising:
a plurality of targets (3) identifying a plurality of injection sites; and
a unique label (5) adjacent to each of the plurality of targets (3).

2. The injection site indicator (1) according to claim 1, further comprising:
an alignment feature corresponding to an anatomical feature.

3. The injection site indicator (1) according to claim 2, wherein the alignment feature includes an eye hole (2).

4. The injection site indicator (1) according to claims 1 or 2, wherein the plurality of targets (3) is arranged in a predetermined pattern relative to the alignment feature.

5. The injection site indicator (1, 6) according to any of the preceding claims, wherein the label (5) includes at least one of a number, a time, a day, a month, a year, a color, text, a graphic and a type of medicament.

6. The injection site indicator (1, 6) according to any of the preceding claims, wherein a given target (3) of the plurality of targets (3) includes a first state indicating that an injection has not been administered for the given target (3) and a second state indicating that an injection has been administered for the given target (3).

7. The injection site indicator (1) according to any of the preceding claims, wherein the plurality of targets (3) is dye packs or perforated elements.

8. The injection site indicator (1, 6) according to any of the preceding claims, wherein the indicator (1, 6) is an eye mask or a chart.
